# EUROPÄISCHE PATENTSCHRIFT

(11) **EP 1 713 763 B1**
(45) Veröffentlichungstag und Bekanntmachung des Hinweises auf die Patenterteilung: **18.05.2011**
(21) Anmeldenummer: 05700951.6
(22) Anmeldetag: 15.01.2005
(51) Int. Cl.: C07C 253/20, C07C 253/22, C07C 291/10, C07C 255/50

(54) **VERFAHREN ZUR HERSTELLUNG VON NITRILEN UND ISONITRILEN DURCH DEHYDRATISIERUNGSREAKTIONEN MIT PROPANPHOSPHONSAEUREANHYDRIDEN**
METHOD FOR PRODUCING NITRILES AND ISONITRILES BY USING DEHYDRATION REACTIONS WITH PROPANEPHOSPHONIC ACID ANHYDRIDES
PROCEDES POUR PREPARER DES NITRILES ET DES ISONITRILES PAR DESHYDRATATION AVEC DES ANHYDRIDES D'ACIDE PROPANE-PHOSPHONIQUE

(30) Priorität: 26.01.2004 DE 102004003953
(43) Veröffentlichungstag der Anmeldung: 25.10.2006
(73) Patentinhaber: Archimica GmbH, 65929 Frankfurt am Main (DE)
(72) Erfinder: MEUDT, Andreas, 65719 Hofheim (DE); SCHERER, Stefan, 64347 Griesheim (DE); NERDINGER, Sven, 83088 Kiefersfelden (DE)
(74) Vertreter: Schweitzer, Klaus
(86) Internationale Anmeldenummer: PCT/EP2005/000361
(87) Internationale Veröffentlichungsnummer: WO 2005/070879

(56) Entgegenhaltungen:
- US-A- 2 200 734
- J. MARCH: "Advanced Organic Chemistry, second edition" 1977, MCGRAW-HILL NTERNATIONAL BOOK COMPANY , AUCKLAND , XP002331027 Seite 953, Zeile 1 - Seite 954, Zeile 3
- WISSMANN H ET AL: "NEUE PEPTIDSYNTHESE" ANGEWANDTE CHEMIE, VCH VERLAGSGESELLSCHAFT, WEINHEIM, DE, Bd. 92, Nr. 2, 1980, Seiten 129-130, XP000195949 ISSN: 0044-8249

## Beschreibung

Nitrile und Isonitrile sind bedeutsame und äußerst vielseitig verwendbare Zwischenprodukte in der organischen Synthese, Beide Verbindungsklassen zeigen eine hohe Reaktivität der C,N-Mehrfachbindung, wodurch zahllose Heterocarbonyl-Reaktionen ermöglicht werden. Die Bedeutung in der modernen organischen Synthese wird nur durch Limitierungen der Zugänglichkeit dieser Verbindungsklassen eingeschränkt. Standardverfahren zur Herstellung von Nitrilen sind Dehydratisierungen von Carbonsäureamiden, wobei zahllose Reagenzien wie beispielsweise POCl3 eingesetzt werden können, Siehe hierzu beispielsweise J. MARCH, Advanced Organic Chemistry, 2nd edition 1977, MCGRAW-HILL INTERNATIONAL BOOK COMPANY, Auckland, Seiten 963-964. Isonitrile sind analog durch Dehydratisierung von Formamiden mit POCl3 zugänglich und können beispielsweise in Ugi-Mehrkomponentenreaktionen Einsatz finden,

In der modernen organischen Synthese nimmt die Bedeutung chemo-, regio- und stereoselektiver Reagenzien explosionsartig zu, Will man beispielsweise in einem komplexen Molekül mit zahlreichen, teilweise nur geringe Reaktivitätsunterschlede aufweisenden funktionellen Gruppen eine bestimmte Säuregruppe in ein Amid überführen, ohne dabei andere Gruppen zu beeinflussen (z.B. Epimerisierung chiraler Funktionalitäten), können unselektive Methoden wie SOCl2 keine Abwendung mehr finden. Ein hochselektives Reagenz zur äußerst selektiven Amidbindungsknüpfung, das selbst in Oligopeptiden ausgezeichnete Selektivitäten und Ausbeuten ohne Epimerisierung erbringt, ist Propanphosphonsäureanhydrid (T3P®). Dieses Reagenz ist in verschiedenen Lösungsmitteln kommerziell erhältlich und bequem einsetzbar. Die Performance dieses Reagenzes ist so hoch, dass das Problem der Knüpfung von Amid- und Peptidbindungen in komplexen Molekülen heute als gelöst betrachtet werden kann (siehe beispielsweise ANGEWANDTE CHEMIE, Bd. 92. Nr. 2, 1980, Seiten 129-130).

Eine vergleichbare Problemlösung für die Überführung von Carbonsäuren, Ammoniumsalzen von Carbonsäuren und Carbonsäureamide in die entsprechenden Nitrile und die Überführung von Formamiden in die entsprechenden Isonitrile fehlte bisher. Die bekannten Reagenzien können zwar die gewünschten Transformationen bewerkstelligen, dabei werden aber oft andere Gruppierungen ebenfalls beeinflusst. In vielen Fällen werden durch die drastischen erforderlichen Bedingungen selbst entfernt stehende Stereozentren epimerisiert.

Es wäre daher sehr wünschenswert, ein Verfahren zu haben, das Carbonsäuren, Ammoniumsalze von Carbonsäuren und Carbonsäureamide durch Dehydratisierung in die entsprechenden Nitrile sowie Formamide durch Dehydratisierung in die entsprechenden Isonitrile überführen kann, dabei aber gleichzeitig sehr hohe Chemoselektivitäten aufweist, und zusätzlich in wirtschaftlich nutzbaren Verfahren einsetzbar ist. Die bekannten Reagenzien lösen dieses Problem nicht, wie an einigen Beispielen demonstriert werden soll: POCl3 in Kombination mit Basen kann die genannten Reaktionen zwar bewerkstelligen, allerdings reagiert fast jede mögliche funktionelle Gruppe ebenfalls mit diesem Reagenz. Mit Dicyclohexylcarbodiimid (DCC) kann man ebenfalls die gewünschte Transformation in Nitrile durchführen, allerdings treten hierbei häufig partielle Epimerisierungen auf; schlimmer noch sind häufig aber die Eigenschaften des als Folgeprodukt gebildeten Dicyclohexylharnstoffs, der oft kaum oder nur durch chromatograpische Trennungen vom Produkt getrennt werden kann. Die Verwendung von wasserlöslichen DCC-Derivaten ist meist durch deren sehr hohen Preis und schwierige Zugänglichkeit nicht wirtschaftlich durchführbar.

Überraschenderweise wurde gefunden, dass cyclische 2,4,6-substituierte 1,3,5,2,4,6-trioxatriphosphinan-2,4,6-trioxide alle diese Probleme lösen und ein ideales und hochselektives Reagenz zur Überführung von Carbonsäuren, Ammoniumsalzen von Carbonsäuren und Carbonsäureamiden durch Dehydratisierung in die entsprechenden Nitrile sowie von Formamiden durch Dehydratisierung in die entsprechenden Isonitrile ist, wobei gleichzeitig die gewünschte Epimerisierungsfreiheit und maximale Regio- und Stereoselektivität beobachtet wird.

Die vorliegende Erfindung betrifft somit ein hochselektives Verfahren zur Herstellung von a.) Nitrilen der Formel (II), sowie b.) Isonitrilen der Formel (III)

R-C≡ N (II)

R-N≡ C (III)

durch Umsetzung von
a) Carbonsäureamiden (RCO-NH2), Ammoniumsalzen von Carbonsäuren (RCOO-NH4+) oder Carbonsäuren in Gegenwart von Ammoniak oder Ammoniumsalzen (RCOOH +NH3, RCOOH + NH4+) oder
b) Formamiden (H-CO-NHR) oder Mischungen von Aminen mit Ameisensäure, mit cyclischen Phosphonsäureanhydriden unter Abspaltung von Wasser, bei einer Temperatur im Bereich von -30 bis + 120°C,
wobei R für einen beliebig substituierten, linearen oder verzweigten C₁-C₈-Alkylrest, einen C₃-C₁₀-Cycloalkyl-, Alkenyl-, Alkinyl- oder einen Aryl- oder Heteroarylrest steht.

In einer bevorzugten erfindungsgemäßen Ausführungsform ist das cyclische Phosphonsäureanhydrid eine Verbindung der Formel (I), 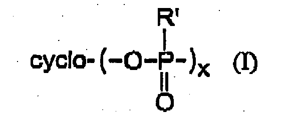 worin x = 3, 4 oder 5,
und R¹ unabhängig voneinander für offenkettige oder verzweigt, gesättigte oder ungesättigte, geradkettige C₁ bis C₁₆-Alkylreste, insbesondere einen C₂ bis C₁₂-Alkylrest, oder cyclische C₃ bis C₁₆-Alkyreste oder für Aryl oder Heteroaryl steht.

In einer besonders bevorzugten erfindungsgemäßen Ausführungsform ist das cyclische Phosphonsäureanhydrid ein 2,4,6-R'-substituiertes 1,3,5,2,4,6-trioxatriphosphinan-2,4,6-trioxid, worin R¹ unabhängig voneinander für offenkettige oder verzweigte, gesättigte oder ungesättigte, geradkettige C₁ bis C₁₆-Alkylreste, insbesondere einen C₂ bis C₁₂-Alkylrest, oder cyclische C₃ bis C₁₆-Alkylreste oder für Aryl oder Heteroaryl steht.

Besonders bevorzugt werden Phosphonsäureanhydride der Formel (I), in denen R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht, ganz besonders bevorzugt Propanphosphonsäureanhydrid (T3P).

Die Dehydratisierung zu Nitrilen (II) und Isonitrilen (III) kann dabei im allgemeinen bei Temperaturen im Bereich von -30 bis +120°C durchgeführt werden, bevorzugt sind Temperaturen im Bereich von +30 bis +70°C, wobei tiefere Temperaturen im allgemeinen mit höheren Selektivitäten korreliert sind. Die Reaktionsdauer ist abhängig von der angewandten Temperatur und beträgt im allgemeinen 1 bis 12 Stunden, insbesondere 3 bis 6 Stunden.

Das cyclische Phosphonsäureanhydrid kann dem Reaktionsmedium entweder als Schmelze oder als flüssige Mischung gelöst in einem Lösungsmittel zugegeben werden.
Geeignete Lösungsmittel sind dabei solche, die keine Nebenreaktionen mit dem Phosphonsäureanhydrid ergeben, dies sind alle aprotischen organischen Lösungsmittel, wie z.B. Ligroin, Butan, Pentan, Hexan, Heptan, Octan, Cyclopentan, Cyclohexan, Cycloheptan, Cyclooctan, Dichlormethan, Chloroform, Tetrachlorkohlenstoff, 1,2-Dichlorethan, 1,1,2,2-Tetrachlorethan, Methylacetat, Ethylacetat, Propylacetat, Butylacetat, Dimethylformamid, Diethylformamid, Dimethylacetamid, Diethylacetamid, Diethylether, Düsopropylether, tert-Butyl-Methylether, THF, Dioxan, Acetonitril oder Gemische aus diesen, besonders bevorzugt sind Dichlormethan, Chloroform, Ethylacetat, Propylacetat, Butylacetat, Dimethylformamid, Diethylformamid, Dimethylacetamid, Diethylacetamid, Diisopropylether, tert-Butyl-Methylether, THF, Dioxan, Acetonitril oder Gemische aus diesen, ganz besonders bevorzugt werden Dichlormethan, Chloroform, Ethylacetat, Butylacetat, Dimethylformamid, Dimethylacetamid, , tert-Butyl-Methylether, THF, Dioxan, Acetonitril oder Gemische aus diesen, insbesondere bevorzugt sind THF, Ethylacetat oder Butylacetat.

Die Zugabe des Phosphonsäureanhydrids erfolgt mindestens stöchiometrisch in Bezug auf die Ausgangsverbindung, kann aber auch überstöchiometrisch sein, beispielsweise im Verhältnis 1:2.

Die Umsetzungen werden bevorzugt so durchgeführt, dass das entsprechende Amid oder Formamid in einem Lösungsmittel vorgelegt wird, dann auf die Reaktionstemperatur erwärmt und anschließend durch Zudosieren des Phonphonsäureanhydrids als Schmelze oder Lösung in einem der vorstehend genannten Lösungsmittel dieses in das gewünschte Nitril oder Isonitril überführt wird.

Die Isolierung des Reaktionsproduktes erfolgt bevorzugt durch Hydrolyse und einfache Phasentrennung, da die Folgeprodukte der Phosphonsäureanhydride allgemein sehr gut wasserlöslich sind. Je nach Natur des zu isolierenden Produkts können dabei auch Nachextraktionen erforderlich sein. Das gebildete Phosphonsäureanhydrid-Folgeprodukt stört Folgereaktionen oft nicht, so dass auch der direkte Einsatz der erhaltenen Reaktionslösungen oft sehr gute Ergebnisse bringt.
Soll ein Amin in ein Isonitril überführt werden, so kann dies sehr elegant durch Reaktion des Amins (H-CO-NHR) mit Ameisensäure und dem Phosphonsäureanhydrid geschehen, wobei zunächst das Formamid gebildet wird, das abschließend zum Isonitril dehydratisiert wird. Es ist ebenfalls möglich, ein Amin nach Verfahren des Standes der Technik zunächst in Formamide zu überführen (z. B. mit Ameisensäureestern) und diese dann mit den cyclischen Phosphonsäureanhydriden zu Isonitrilen zu dehydratisieren.

Soll ein Ammoniumsalz einer Carbonsäure (RCOO-NH4+) in ein Nitril überführt werden, so kann dies durch einfaches Erwärmen mit dem Phosphonsäureanhydrid analog dem oben beschriebenen Verfahren durchgeführt werden. Hieraus ergibt sich auch ein sehr elegantes und ebenfalls äußerst selektives Verfahren zur direkten Überführung von Carbonsäuren in Nitrile dergestalt, dass man ein beliebiges Ammoniumsalz, bevorzugt Ammoniumchlorid oder Ammoniumsulfat, zur Carbonsäure dazugibt und anschließend in Gegenwart einer Base mit einem Phosphonsäureanhydrid umsetzt.
Geeignete Basen sind beispielsweise tertiäre Amine wie Triethylamin, Tripropylamin, Benzyldimethylamin, N,N-Dimethylanilin oder Pyridin.
Die Base wird üblicherweise in einem Verhältnis von 1 bis 2 Äquivalenten, vorzugsweise 1 bis 1,2 Äquivalenten, bezogen auf die Carbonsäure zugegeben.

Das Verfahren kann auch so durchgeführt werden, dass eine Lösung oder Suspension der umzuwandelnden Carbonsäure in Kohlenwasserstoffen oder Estern wie Ethyl- oder Butylacetat mit mindestens einem Äquivalent Ammoniakgas gesättigt und nachfolgend mit dem Phosphonsäureanhydrid behandelt wird.

Alle benannten Verfahrensweisen zeichnen sich durch sehr gute Ausbeuten (typisch 90-100 %, insbesondere > 95 %) bei gleichzeitiger Abwesenheit von Nebenreaktionen sowie von Epimerisierungen aus. Die Selektivitäten der erfindungsgemäßen Reaktion liegen im Bereich von 97-100 %, insbesondere 99-100 %.

Das erfindungsgemäße Verfahren soll durch die nachfolgenden Beispiele erläutert werden

### Beispiel 1: Dehydratisierung von Benzamid zu Benzonitril

1 mol Benzamid wird in 150 mL Ethylacetat vorgelegt und auf 45°C erhitzt. 1,2 mol T3P-Lösung in Ethylacetat (50 % w/w) werden im Laufe einer Stunde zudosiert, anschließend wird weitere drei Stunden bei dieser Temperatur nachgerührt. Zu dieser Zeit zeigte das Reaktions-GC einen Umsatz von 100 % an. Nach Abkühlen auf Raumtemperatur wurden 180 mL Wasser zugeben und die Phasen getrennt. Die organische Phase wurde destilliert. Die isolierte Ausbeute dieser Reaktion betrug 96 %.

### Beispiel 2: Dehydratisierung von N-Formyl-o-tolylamin zu o-Tolylisonitril

0,1 mol N-Formyl-o-tolylamin wird in 50 mL Ethylacetat vorgelegt und auf 55°C erhitzt. 0,12 mol T3P-Lösung in Ethylacetat (50 % w/w) werden im Laufe einer Stunde zudosiert, anschließend wird weitere zwei Stunden bei dieser Temperatur nachgerührt. Zu dieser Zeit zeigte das Reaktions-GC einen Umsatz von > 99 % an. Nach Abkühlen auf 0 °C wurden 25 mL Wasser zugeben und die Phasen getrennt. Nach Abkondensieren des Lösungsmittels verblieb das Isonitril in einer Ausbeute von 97 %, HPLC-Reinheit 98 % (a/a).

### Beispiel 3: Dehydratisierung von Ammoniumbenzoat

1 mol Ammoniumbenzoat wird in 180 mL Butylacetat vorgelegt und auf 45°C erhitzt. 1,2 mol T3P-Lösung in Butylacetat (50 % w/w) werden im Laufe einer Stunde zudosiert, anschließend wird weitere sechs Stunden bei dieser Temperatur nachgerührt. Zu dieser Zeit zeigte das Reaktions-GC einen Umsatz von > 99 % an. Nach Abkühlen auf Raumtemperatur wurden 140 mL Wasser zugeben und die Phasen getrennt. Die organische Phase wurde destilliert. Die isolierte Ausbeute dieser Reaktion betrug 94 %.

### Beispiel 4: Benzonitril aus Benzoesäure

1 mol Benzoesäure, 1,05 mol Ammoniumchlorid und 1,08 mol Triethylamin werden in 100 mL Ethylacetat suspendiert und auf 65°C erhitzt. 1,2 mol T3P-Lösung in Ethylacetat (50 % w/w) werden im Laufe einer Stunde zudosiert, anschließend wird weitere drei Stunden bei dieser Temperatur nachgerührt. Zu dieser Zeit zeigte das Reaktions-GC einen Umsatz von 99,8 % an. Nach Abkühlen auf Raumtemperatur wurden 150 mL Wasser zugeben und die Phasen getrennt. Die organische Phase wurde destilliert. Die isolierte Ausbeute dieser Reaktion betrug 94 %.

### Beispiel 5: Dehydratisierung von N-Formyl-L-Phenylalanin-Methylester

1 mmol N-Formyl-L-Phenylalanin-Methylester wird in 10 mL Ethylacetat vorgelegt und auf 28°C erhitzt. 1,1 mmol T3P-Lösung in Ethylacetat (50 % w/w) werden im Laufe einer Stunde zudosiert, anschließend wird weitere drei Stunden bei dieser Temperatur nachgerührt. Zu dieser Zeit zeigte das Reaktions-HPLC den kompletten Verbrauch des Edukts an. Nach Abkühlen auf 0°C wurden 5 mL Wasser zugeben und die Phasen getrennt. Nach vorsichtigem Abkondensieren des Lösungsmittels bei max. 30°C verblieb das gewünschte chirale Isonitril als farbloses Öl, Rohausbeute 99 %. Die Umsetzung des Isonitrils sollte alsbald geschehen, da ansonsten rasche Racemisierung eintritt.

## Patentansprüche

1. Verfahren zur Herstellung von a) Nitrilen der Formel (II) und b) Isonitrilen der Formel (III)
R-C ≡ N (II)
R-N ≡ C (III)
durch Umsetzung von
a) Carbonsäureamiden (RCO-NH2), Ammoniumsalzen von Carbonsäuren (RCOO-NH4+) oder Carbonsäuren in Gegenwart von Ammoniak oder Ammoniumsalzen (RCOOH +NH3, RCOOH + NH4+) oder
b) Formamiden (H-CO-NHR) oder Mischungen von Aminen mit Ameisensäure,
mit cyclischen Phosphonsäureanhydriden unter Abspaltung von Wasser, bei einer Temperatur im Bereich von -30 bis + 120°C,
wobei R für einen beliebig substituierten linearen oder verzweigten C₁-C₈-Alkylrest, einen C₃-C₁₀-Cycloalkyl-, Alkenyl-, Alkinyl- oder einen Aryl- oder Heteroarylrest steht.

2. Verfahren nach Anspruch 1, **dadurch gekennzeichnet, dass** das cyclische Phosphonsäureanhydrid eine Verbindung der Formel (I) ist, 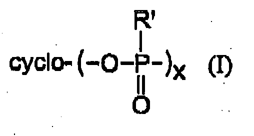 worin x = 3, 4 oder 5 ist und
R' unabhängig voneinander für offenkettige oder verzweigte, gesättigte oder ungesättigte, geradkettige C₁ bis C₁₆-Alkylreste oder cyclische C₃ bis C₁₆-Alkylreste oder für Aryl oder Heteroaryl steht,

3. Verfahren nach Anspruch 1, **dadurch gekennzeichnet, dass** das cyclische Phosphonsäureanhydrid 2,4,6-R'-substituiertes 1,3,5,2,4,6-trioxatriphosphinan-2,4,6-trioxid ist.
worin R' unabhängig voneinander für offenkettige oder verzweigte, gesättigte oder ungesättigte, geradkettige C₁ bis C₁₆-Alkylreste oder cyclische C₃ bis C₁₆-Alkylreste oder für Aryl oder Heteroaryl steht.

4. Verfahren nach Anspruch 2 oder 3, **dadurch gekennzeichnet, dass** R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht.

5. Verfahren nach Anspruch 2 oder 3, **dadurch gekennzeichnet, dass** R' für einen Ethyl-, Propyl- und/oder Butyl-Rest steht,

6. Verfahren nach Anspruch 2 oder 3, **dadurch gekennzeichnet, dass** das cyclische Phosphonsäureanhydrid Propanphosphonsäureanhydrid ist

7. Verfahren nach mindestens einem der vorhergehenden Ansprüche, **dadurch gekennzeichnet, dass** das cyclische Phosphonsäureanhydrid entweder als Schmelze oder gelöst in einem Lösungsmittel der Amid- oder Formamid- enthaltenden Reaktionslösung zugegeben wird.

8. Verfahren nach Anspruch 7, **dadurch gekennzeichnet, dass** das cyclische Phosphonsäureanhydrid in einem aprotischen Lösungsmittel zugegeben wird.

9. Verfahren nach Anspruch 7, **dadurch gekennzeichnet, dass** das cyclische Phosphonsäureanhydrid in einem aprotischen Lösungsmittel in einem Verhältnis von 1:1 bis 1:2 zugegeben wird.

10. Verfahren nach mindestens einem der vorhergehenden Ansprüche, **dadurch gekennzeichnet, dass** die Reaktionslösung nach Zugabe des Phosphonsäureanhydrids auf die Reaktionstemperatur erhitzt wird.

11. Verfahren nach mindestens einem der vorhergehenden Ansprüche, **dadurch gekennzeichnet, dass** bei der Herstellung von Nitrilen ein Ammoniumsalz mit einer Carbonsäure (R-COOH) in Gegenwart einer Base mit dem Phosphonsäureanhydrid umgesetzt wird.

12. Verfahren nach Anspruch 11, **dadurch gekennzeichnet, dass** als Base Triethylamin, Tripropylamin, Benzyldimethylamin, N,N-Dimethylanilin oder Pyridin eingesetzt wird.

## Claims

1. A process for preparing a) nitriles of the formula (II) and b) isonitriles of the formula (III)
R-C≡N (II)
R-N≡C (III)
by reacting
a) carboxamides (RCO-NH2), ammonium salts of carboxylic acids (RCOO-NH4+) or carboxylic acids in the presence of ammonia or ammonium salts (RCOOH + NH3, RCOOH + NH4+) or
b) formamides (H-CO-NHR) or mixtures of amines with formic acid, with cyclic phosphonic anhydrides with elimination of water at a temperature in the range from -30 to +120 °C,
where R may have any substitution and is a linear or branched C₁-C₈-alkyl radical, a C₃-C₁₀-cycloalkyl, alkenyl, alkynyl or an aryl or heteroaryl radical.

2. The process as claimed in claim 1, wherein the cyclic phosphonic anhydride is a compound of the formula (I) 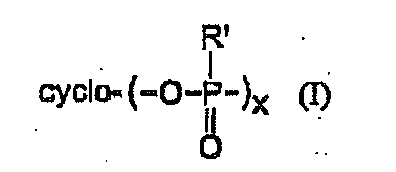 where x = 3, 4 or 5 and
R' are each independently open-chain or branched, saturated or unsaturated, straight-chain C₁ to C₁₆-alkyl radicals or cyclic C₃ to C₁₆-alkyl radicals, or aryl or heteroaryl.

3. The process as claimed in claim 1, wherein the cyclic phosphonic anhydride is 2,4,6-R'-substituted 1,3,5,2,4,6-trioxatriphosphinane 2,4,6-trioxide in which
R' are each independently open-chain or branched, saturated or unsaturated, straight-chain C₁ to C₁₆-alkyl radicals or cyclic C₃ to C₁₆-alkyl radicals, or aryl or heteroaryl.

4. The process as claimed in claim 2 or 3, wherein R' is a methyl, ethyl, n-propyl, isopropyl, n-butyl, 2-butyl, isobutyl, pentyl, hexyl, in particular an ethyl, propyl, and/or butyl radical.

5. The process as claimed in claim 2 or 3, wherein R' is an ethyl, propyl and/or butyl radical.

6. The process as claimed in claim 2 or 3, wherein the cyclic phosphonic anhydride is propanephosphonic anhydride.

7. The process as claimed in at least one of the preceding claims, wherein the cyclic phosphonic anhydride is added to the amide- or formamide-containing reaction solution either as a melt or dissolved in a solvent.

8. The process as claimed in claim 7, wherein the cyclic phosphonic anhydride is added in an aprotic solvent.

9. The process as claimed in claim 7, wherein the cyclic phosphonic anhydride is added in an aprotic solvent in a ratio of from 1:1 to 1:2.

10. The process as claimed in at least one of the preceding claims, wherein the reaction solution is heated to the reaction temperature after addition of the phosphonic anhydride.

11. The process as claimed in at least one of the preceding claims, wherein, in the case of preparation of nitriles, an ammonium salt together with a carboxylic acid (R-COOH) is reacted with the phosphonic anhydride in the presence of a base.

12. The process as claimed in claim 11, wherein the base used is triethylamine, tripropylamine, benzyldimethylamine, N,N-dimethylaniline or pyridine.

## Revendications

1. Procédés pour la préparation a) de nitriles de formule (II) et b) d'isonitriles de formule (III)
R-C≡N (II)
R-N≡C (III)
par la réaction
a) d'amides d'acides carboxyliques (RCO-NH2), de sels d'ammonium d'acides carboxyliques (RCOO-NH4+) ou d'acides carboxyliques en présence d'ammoniac ou de sels d'ammonium (RCOOH + NH3, RCOOH + NH4+) ou
b) de formamides (H-CO-NHR) ou de mélanges d'amines et d'acide formique ;
avec des anhydrides d'acides phosphoniques cycliques, par une élimination d'eau à une température dans l'intervalle de -30 à +120 °C,
où R représente, au choix, un radical alkyle en C₁-C₈-substitué linéaire ou branché, un radical cycloalkyle en C₃-C₁₀-, un radical alcényle, un radical alcynyle ou un radical aryle ou hétéroaryle.

2. Procédés selon la revendication 1 **caractérisés en ce que** l'anhydride d'acide phosphonique cyclique est un composé de formule (I), 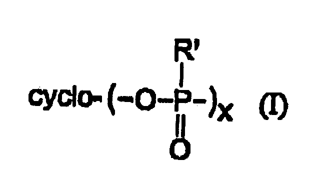 où x est = à 3, 4 ou 5 et
R' représente, indépendamment les uns des autres, des radicaux alkyles linéaires de C₁ à C₁₆ à chaîne ouverte ou branchée, saturés ou insaturés, ou des radicaux alkyles cycliques de C₃ à C₁₆ ou des radicaux aryles ou hétéroaryles.

3. Procédés selon la revendication 1 **caractérisés en ce que** l'anhydride d'acide phosphonique cyclique est du 1,3,5,2,4,6-trioxatriphosphinan-2,4,6-trioxyde substitué R' en 2,4,6.
où R' représente, indépendamment les uns des autres, des radicaux alkyles linéaires de C₁ à C₁₆ à chaîne ouverte ou branchée, saturés ou insaturés, ou des radicaux alkyles cycliques de C₃ à C₁₆ ou des radicaux aryles ou hétéroaryles.

4. Procédés selon les revendications 2 ou 3 **caractérisés en ce que** R' représente un radical méthyle, éthyle, n-propyle, isopropyle, n-butyle, 2-butyle, isobutyle, pentyle, hexyle, en particulier un radical éthyle, propyle et/ou butyle.

5. Procédés selon les revendications 2 ou 3 **caractérisés en ce que** R' représente un radical éthyle, propyle et/ou butyle.

6. Procédés selon les revendications 2 ou 3 **caractérisés en ce que** l'anhydride de l'acide phosphonique cyclique est l'anhydride de l'acide propane-phosphonique.

7. Procédés selon au moins l'une des revendications précédentes **caractérisés en ce que** l'anhydride de l'acide phosphonique cyclique est ajouté à la solution réactionnelle contenant l'amide ou le formamide, soit à l'état fondu, soit dissout dans un solvant.

8. Procédés selon la revendication 7 **caractérisés en ce que** l'anhydride de l'acide phosphonique cyclique est ajouté dans un solvant aprotique.

9. Procédés selon la revendication 7 **caractérisés en ce que** l'anhydride de l'acide phosphonique cyclique est ajouté dans un solvant aprotique à un ratio de 1 : 1 à 1 : 12.

10. Procédés selon au moins l'une des revendications précédentes **caractérisés en ce que** la solution réactionnelle est réchauffée à la température réactionnelle après l'ajout de l'anhydride de l'acide phosphonique cyclique.

11. Procédés selon au moins l'une des revendications précédentes **caractérisés en ce que**, dans la fabrication de nitriles, un sel d'ammonium est mis à réagir avec un acide carboxylique (R-COOH) en présence d'une base avec l'anhydride de l'acide phosphonique.

12. Procédés selon la revendication 11 **caractérisés en ce qu'**on utilise en tant que base de la triéthylamine, de la tripropylamine, de la benzyldiméthylamine, de la N,N-diméthylaniline ou de la pyridine.
